(19) Europäisches Patentamt
European Patent Office
Office européen des brevets

(11) Numéro de publication: **0 074 863**
**B1**

(12) # FASCICULE DE BREVET EUROPEEN

(45) Date de publication du fascicule du brevet: **26.02.86**

(51) Int. Cl.⁴: **C 07 D 237/20**, C 07 D 409/04

(21) Numéro de dépôt: **82401424.5**

(22) Date de dépôt: **30.07.82**

(54) **Nouveau dérivé de la pyridazine actif sur le système nerveux central.**

(30) Priorité: **07.08.81 FR 8115380**

(43) Date de publication de la demande:
**23.03.83 Bulletin 83/12**

(45) Mention de la délivrance du brevet:
**26.02.86 Bulletin 86/9**

(84) Etats contractants désignés:
**AT BE CH DE FR GB IT LI LU NL SE**

(56) Documents cités:
**CHEMICAL ABSTRACTS, vol.76, 1972, résumé no.41974h, page 22, Columbus, Ohio (US) M.R. ORNELLAS: "Biochemical studies of cerebral subfractions after chronic administration of 4-methyl-3-(2-(morpholino)ethylamino)-6-phenylpyridazine hydrochloride, AG 620"**
**CHEMICAL ABSTRACTS, vol.73, 1970, résumé no.12845z, page 199, Columbus, Ohio (US) M.R. ORNELLAS et al.: "Pharmacological interpretation of the energy metabolism of rat brain in vivo and in vitro in connection with a study on some pyridazines"**

(73) Titulaire: **SANOFI, société anonyme, 40, Avenue George V, F-75008 Paris (FR)**

(72) Inventeur: **Wermuth, Camille, 3 rue de la Côte d'Azur, F-67100 Strasbourg (FR)**
Inventeur: **Kan, Jean-Paul, 25 Lotissement l'Olivette, F-34170 Clapiers (FR)**
Inventeur: **Bizière, Kathleen, 6 Lotissement Rayons d'Oc, F-34170 Clapiers (FR)**

(74) Mandataire: **Combe, André et al, CABINET BEAU DE LOMENIE 55 rue d'Amsterdam, F-75008 Paris (FR)**

**EP 0 074 863 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne un nouveau dérivé de la phényl-5 amino-3 pyridazine un procédé pour sa préparation et des compositions pharmaceutiques.

Depuis de nombreuses années des dérivés de la pyridazine ont été proposés en tant que médicaments. Dans un grand nombre de cas il s'agit de substances actives sur le système cardiovasculaire et présentant en particulier un effet hypotenseur ou vasodilatateur. Plus rarement on a mentionné parmi les dérivés de la pyridazine une action antiinflammatoire et analgésique. Enfin le brevet français n° 2 141 697 décrit une famille de produits répondant à la formule générale:

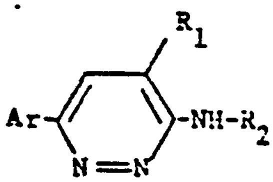

où:

. $R_1$ représente l'hydrogène ou un groupe alkyle inférieur;

. Ar représente un reste aromatique;

. $R_2$ désigne un groupe $-(CH_2)_n$

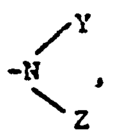

dans lequel n = 2 ou 3 et Y et Z représentent un groupe alkyle inférieur ou bien

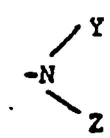 constitue

un radical hétérocyclique.

Ces composés sont caractérisés par une action psychotrope de type psychostimulante.

Une étude ultérieure du composé où $R_1 = CH_3$ Ar = phényle et $R_2 = -CH_2CH_2-$

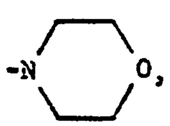

qui a reçu la Dénomination Commune Internationale 'Minaprine", a permis de montrer que l'activité pharmacologique de la minaprine est caractérisée par un mécanisme d'action noradréngergique, dopaminergique et sérotoninergique, voir notamment K. BIZIERE et al., Arzneimittel Forschung 32 (II) n° 8 (1982).

Le métanisme d'action dopaminergique a suggéré l'utilisation de la minaprine dans le traitement de l'hyperkinésie chez l'enfant (brevet US 4 232 020) et notamment l'action noradrénergique a suggéré l'utilisation de la minaprine comme intidépresseur.

Les trois types de mécanisme d'action conduisent cependant à tonsidérer la minaprine comme un produit ayant une activité multiple peu sélective.

On a maintenant trouvé qu'un nouveau dérivé de la phényl -5 amino-3 pyridazine possède une activité antidépressive sélective du type noradrénergique.

La présente invention a donc pour objet selon un de ses aspects un dérivé de la pyridazine à savoir la (morpholino-2 éthyl-amino)-3 phényl-5 pyridazine répondant à la formule I

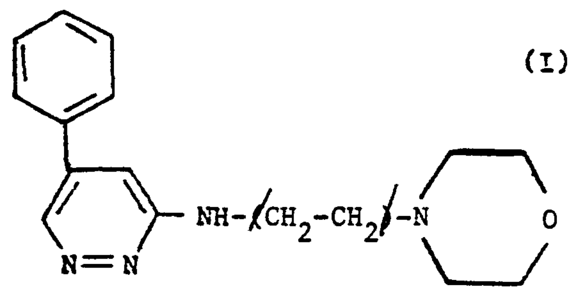

(I)

ainsi que ses sels pharmaceutiquement acceptables.

Selon un autre de ses aspects, l'invention concerne un procédé pour la préparation du composé de formule cidessus, ledit procédé étant caractérisé en ce que l'on traite la chloro- 3 phényl-5 pyridazine de formule II

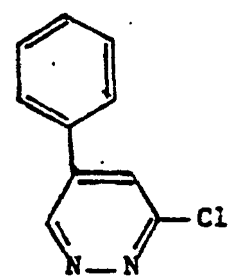

(II)

avec la morpholino-2 éthylamine de formule III

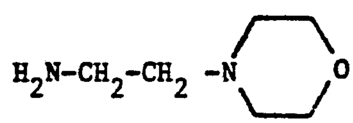

(III)

dans un solvant organique à une température comprisc entre 50°C et la température d'ébullition du solvant employé et l'on transforme éventuellement le produit ainsi obtenu en ses sels pharmaceutiquement acceptables.

La réaction entre le dérivé chloré II et l'amine III

s'effectue en général par chauffage au sein d'un solvant convenable tel qu'un alcool le plus souvent à la température d'ébullition du solvant. La durée de la réaction varie de quelques heures à plusieurs jours suivant la température et la nature des réactifs mis en jeu. Lorsque la réaction s'avère trop lente elle peut être catalysée par addition d'une petite quantité de poudre de cuivre.

On effectue de préférence la réaction en présence d'un accepteur d'hydracide destiné à fixer l'acide chlorhydrique formé; le plus souvent on utilise comme tel un excès de l'amine III.

L'isolement du composé I s'effectue par reprise dans l'eau et extraction par un solvant convenable tel que l'acétate d'éthyle.

Le composé I peut être salifié de façon habituelle par action de l'acide sur une solution chaude de la base le solvant étant choisi de façon que le sel cristallise par refroidissement.

La chloro-3 phényl-5 pyridazine utilisée comme produit départ est obtenue à partir de la 2 H pyridazone -3 correspondante par action d'un exces d'oxychlorure de phosphore. La 2 H pyridazone -3

peut être obtenue par des procédés connus tels que l'action de l'hydrazine sur des acides cétoniques ou des dérivés activés de ceux-ci.

Le nouveau composé de formule I ci-dessus ainsi que ses sels pharmaceutiquement acceptables possedent des propriétés Pharmacologiques intéressantes et différentes de celles des composés décrits dans le brevet français n° 2 141 697 notamment de la minaprine.

La nouvelle phényl-5 amino-3 pyridazine de la présente invention et ses sels antagonisent la ptôse à la réserpine ce qui témoigne une activité noradrénergique; ils sont inactifs dans le test de la 'despair réaction" et dans celui du 'circling" ce qui suggère l'absence d'effet dopaminergique.

Le test de l'antagonisme de-la ptôse induite par la réserpine décrit par GOURET [Journal de Pharmacologie (Paris) 1973 4 (1) 105-128 ] a été réalisé chez la souris femelle CDI (Charles River) pesant 20 ± 1 g. La réserpine entraine un ptôsis 1 heure après son administration intraveineuse; certains antidépresseurs s'opposent à ce ptôsis ce qui représente un indice d'un mécanisme d'action noradrénergique.

La substance à étudier a été administrée i.p. La réserpine est administrée simultanément par voie intraveineuse à la dose de 2 mg/kg. 1 heure après l'administration de réserpine on note le nombre d'animaux ne présentant pas de ptôsis.

Ce test a été réalisé sur des lots de 10 souris les résultats sont exprimés en pourcentage d'animaux ne présentant pas de ptôsis et sont la moyenne de au moins deux expériences.

Le test de la 'despair réaction" a été réalisé chez la souris femelle CDI (Charles River) pesant 18 à 23 g selon la méthode décrite par PORSOLT (Archives Internationales de Pharmacodynamie 1977 229 327-336).

Le principe de ce test est le suivant: lorsqu'une souris est placée dans un récipient étroit rempli d'eau elle se débat puis au bout de 2 à 4 min elle s'immobilise et flotte sur le ventre le dos arrondi les pattes postérieurea ramenées sous le corps et elle ne fait que quelques mouvements nécessaires pour se maintenir la tête hors de l'eau. C'est la réaction dite de désespoir (despair réaction) qui représente un indice d'un mécanisme d'action du type dopaminergique.

Certains psychotropes dopaminergiques allongent le temps pendant lequel la souris se débat.

Le produit à étudier a été administré i.p. 1 heure avant le test. Pour ce test les animaux sont placés dans un récipient étroit (10 X 10 x 10 cm) rempli d'eau sur une hauteur de 6 cm la température de l'eau étant de 24 ± 2°C. Les animaux sont laissés 6 min dans l'eau et on mesure le temps où l'animal reste immobile entre la 2e et la 6e minute. Plus ce temps est court plus la substance est active.

Chaque substance a été étudiée sur un lot de 10 souris. Les résultats sont la moyenne d'au moins deux expériences.

L'activité dopaminomimétique du produit de l'invention a été également étudiée sur les récepteurs dopaminergiques striataux de la souris selon le test du 'circling décrit Par P. PORTAIS et J. COSTENTIN Journal de Pharmacologie (Paris), 7, 251-255 (1976).

La lésion unilatérale des neurones dopaminergiques nigrostriataux induit une hypersensibilité des récepteurs de la dopamine au niveau du striatum. L'asymétrie qui en résulte est révélée par des rotations de l'animal dans le sens contralatéral aux récepteurs les plus intensément stimulés.

Après administration du produit à étudier par voie intrapéritonéale on compte pendant une période de 2 min le nombre de tours effectués par l'animal.

On exprime les résultats sous forme de pourcentage des variations par rapport aux témoins n'ayant pas reçu de produit à étudier.

Le tableau I ci-dessous réunit les données de toxicité aiguë par voie intrapéritonéale chez la souris ainsi que les résultats obtenus dans les trois tests ci-dessus pour le composé de la présente invention le chlorhydrate de (morpholino-2 éthylamino)-3 phényl-5 pyridazine (CM 30 364) et pour la minaprine comme produit de référence.

**TABLEAU I**

| Test \ Composé | CM 30 364 | IMIPRAMINE |
|---|---|---|
| $DL_{50}$ (mg/kg i.p.) | 350 | 63 |
| Antagonisme ptôse réserpine $DE_{50}$ (mg/kg i.p.) | 6 (5-8) | 5 (4-7) |
| Despair reaction | inactif | 10 mg/kg : 35% ** |
| Circling 5,3 mmol/kg i.p. | inactif | - 91% |

De ce tableau, il ressort que le produit de la présente invention possède une activité antidépressive du type noradrénergique du même ordre que celle du composé de référence avec une toxicité très faible beaucoup inférieure à celle du composé de réfrence. En outre ce tableau montre que le produit de la présente invention contrairement au produit de référence est dépourvu d'activité du type dopaminergique.

Le produit de l'invention possède donc des propriétés antidépressives avec un mode d'action plus sélectif que celui du composé de référence.

Ainsi le composé de formule I ci-dessus ainsi que ses sels pharmaceutiquement acceptables peuvent être utilisés dans le traitement des états dépressifs sérieux dans la dépression du vieillard ainsi que dans les troubles de la mémoire et de la sénescence.

Ces produits peuvent être administrés par voie orale ou par voie injectable dans des compositions pharmaceutiques solides ou liquides par exemple sous forme de comprimés gélules granulés suppositoires ou préparations injectables seuls ou mélangés avec un excipient pharmaceutique.

Les compositions pharmaceutiques de l'invention sont présentées de préférence sous forme d'unité de dosage contenant de 0 010 à 150 mg de principe actif.

La posologie peut varier dans de larges proportions en particulier suivant le type et la gravité de l'affection à traiter et suivant le mode d'administration. Par voie orale elle est le plus souvent comprise entre 0 010 g et 0 500 g de principe actif éventuellement répartie en plusieurs prises.

L'exemple suivant est donné à titre d'illustration de la présente invention.

**EXEMPLE 1**

**(Morpholino-2 éthvlamino)-3 phényl-5 pyridazine dichlorhydrate (CM 30 364)**

On chauffe à reflux pendant 12 h le mélange de 8 g de chloro-3 phényl,5 pyridazine et 10 g de morpholino-2 éthylamine dans 80 ml de butanol.

On verse la solution chaude dans 200 ml d'eau, filtre le précipité qu'on lave avec un peu d'éther. On sépare la phase aqueuse et on l'extrait avec de l'éther. On réunit les phases éthérées et on les extrait avec une solution d'acide sulfurique 1 N.

La solution aqueuse est alcalinisée par addition d'une solution de carbonate de sodium à 10%. Après une nuit on essore le solide qu'on recristallise dans un mélange isopropanol-éther isopropylique. F. 121°C.

**Dichlorhydrate:**

A une solution de base (8 g) dans 50 ml d'isopropanol on ajoute 5 5 ml d'acide chlorhydrique concentré. On essore les cristaux et recristellise dans l'isopropanol. F. 250°C.

**EXEMPLE 2**

**COMPRIMES**

CM 30 364 200 mg
Cellulose microcristalline 100 mg
Lactose 197 mg
Stéarate de magnésium 3 mg
500 mg

**Revendications** pour les etats contractants BE,CH,DE,FR,GB,IT,LI,LU, NL SE.

1. (Morpholin-2 éthylamino)-3 phényl-5 pyridazine de formule I

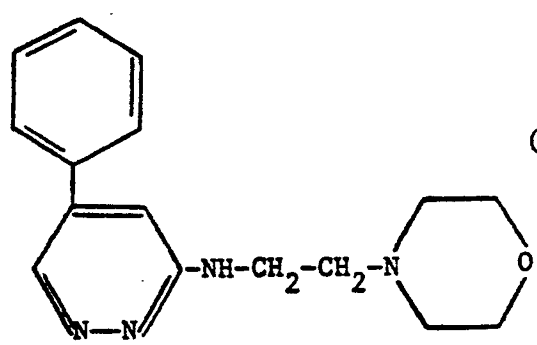

(I)

et ses sels pharmaceutiquement acceptables.

2. Procedé de preparation du produit de formule I, caractérisé en ce que l'on fait réagir la

chloro-3 phényl-5 pyridazine de formule II

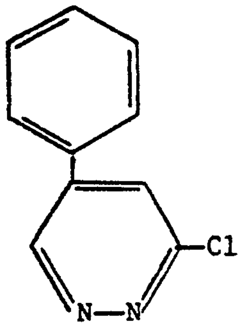

(II)

avec la morpholino-2 éthylamine de formule III

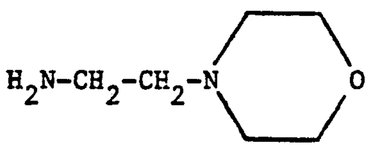

(III)

la réaction étant réalisée dans un solvant organique, tel qu'un alcool, à une température comprise entre 50° C environ et la température d'ébullition dudit solvant, puis on transforme eventuellement le produit ainsi obtenu en l'un de ses sels pharmaceutiquement acceptables.

3. Procede selon la revendicatio, 2, caractérisé en ce que la réaction est effectuée en présence de cuivre utilisé comme catalyseur.

4. Compositions pharmaceutiques utilisables notamment dans le traitement des états dépressifs et dans le traitement des troubles de la mémoire, caractérisées en ce qu'elles contiennent un composé selon la revendication 1.

5. Compositions pharmaceutiques selon la revendication 4, caractérisées en ce qu'elles contiennent de 0,01 à 150 mg d'composé selon la revendication 1.

**Revendications** pour l'etat contractant: AT

1. Procedé pour l'obtention du derive de la(morpholino-2 éthylamino)-3 phenyl-5 pyridazine de formule I

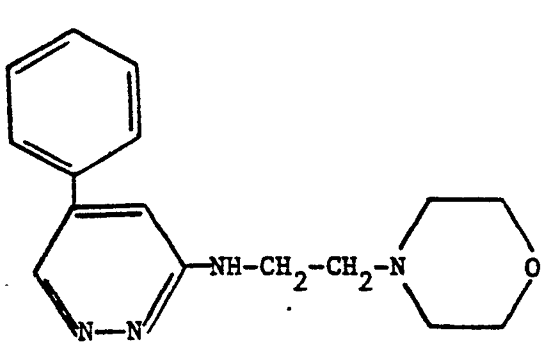

(I)

et de ses sels pharmaceutiquement acceptables, caractérisé en ce que l'on fait réagir la chloro-3 phenyl-5 pyridamine de formule II

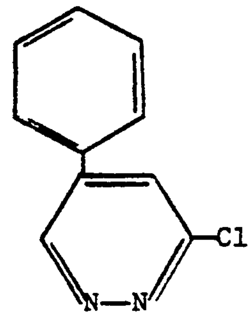

II

avec la morpholino-2 éthylamine de formule III

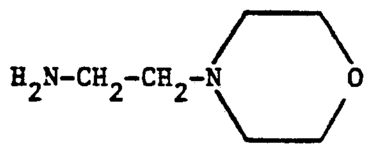

(III)

la réaction étant realisée dans un solvant organique, tel qu'un alcool, à une température comprise entre 50° C environ et la température d'ebullition dudit solvant, puis on transforme eventuellement le produit ainsi obtenu en l'un de ses sels pharmaceutiquement acceptables.

2. Procédé selon la revendication 1, caractérisé en ce que la réaction est effectuée en présence de cuivre utilisé comme catalyseur.

3. Utilisation d'un composé selon la revendication 1, pour la préparation d'un médicament utilisable notamment dans le traitement des états depressifs et dans le traitement des troubles de la mémoire.

4. Utilisation selon la revendication 3, caractérisée en ce que ledit médicament contient de 0,01 à 150 mg d'un composé selon la revendication 1.

**Patentansprüche** für die Vertragsstaaten -BE, CH, DE, FR, GB, IT, LI, LU, NL, SE:

1. 3-(2-Morpholino-äthyl-amino)-5-phenyl-pyridazin der Formel 1

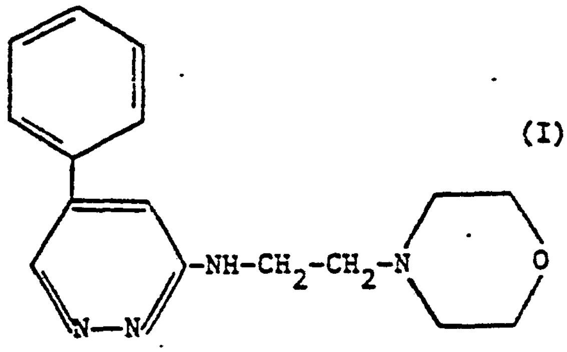

(I)

und seine pharmazeutisch akzeptablen Salze.

2. Verfahren zur Herstellung des Produkts der Formel 1, dadurch gekennzeichnet, daß man 3-Chlor-5-phenyl-pyrida-zin der Formel II

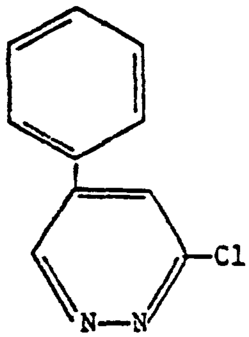

(II)

mit 2-Morpholino-äthylamin der Formel III

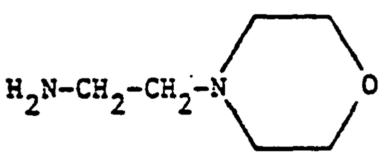

(III)

zur Umsetzung bringt, wobei die Umsetzung in einem organischen Lösungsmittel, wie einem Alkohol, bei einer Temperatur zwischen etwa 50° C und der Kochtemperatur des Lösungsmittels durchgeführt wird, und dann gegebenenfalls das so erhaltene Produkt in eines seiner pharmazeutisch akzeptablen Salze überführt.

3. Verfahren nach Anspruch 2, dadurch gekennzeichnet, daß die Reaktion in Gegenwart von Kupfer als Katalysator durchgeführt wird.

4. Pharmazeutische Zusammensetzungen, die insbesondere bei der Behandlung von depressiven Zuständen und bei der Behandlung von Gedächtnisstörungen nützlich sind, dadurch gekennzeichnet, daß sie eine Verbindung nach Anspruch 1 enthalten.

5. Pharmazeutische Zusammensetzungen nach Anspruch 4, dadurch gekennzeichnet, daß sie 0,01 bis 150 mg der Verbindung nach Anspruch 1 enthalten.

**Patentansprüche** für den Vertragsstaat AT:

1. Verfahren zur Herstellung des Derivats von 3-(2-Mor-pholino-äthyl-amino)-5-phenyl-pyridazin der Formel 1

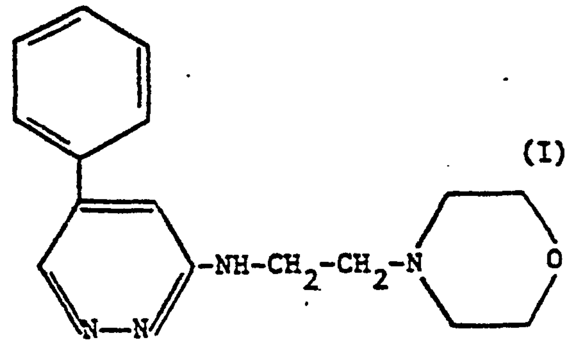

(I)

und seiner pharmazeutisch akzeptablen Salze, dadurch gekennzeichnet, daß man 3-Chlor-5-phenyl-pyridazin der Formel II

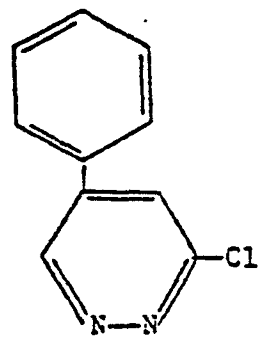

(II)

mit 2-Morpholino-äthylamin der Formel III

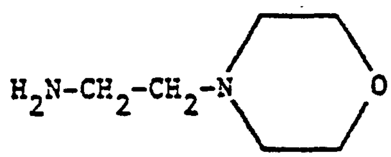

(III)

zur Umsetzung bringt, wobei die Umsetzung in einem organischen Lösungsmittel, wie einem Alkohol, bei einer Temperatur zwischen etwa 50° C und der Kochtemperatur des Lösungsmittels durchgeführt wird, und dann gegebenenfalls das so erhaltene Produkt in eines seiner pharmazeutisch akzeptablen Salze überführt.

2. Verfahren nach Anspruch 1, dadurch gekennzeichnet, daß die Reaktion in Gegenwart von Kupfer als Katalysator durchgeführt wird.

3. Verwendung einer Verbindung nach Anspruch 1 zur Herstellung eines insbesondere bei der Behandlung von depressiven Zuständen und bei der Behandlung von Gedächtnisstörungen nützlichen Medikaments.

4. Verwendung nach Anspruch 3, dadurch gekennzeichnet, daß das Medikament 0,01 bis 150 mg einer Verbindung nach Anspruch 1 enthält.

**Claims** for the contracting states: BE, CH,DE, FR, GB, IT, LI, LU, NL and SE.

1. 3- (2-morpholino ethylamino) 5-phenyl pyridazine of formula I

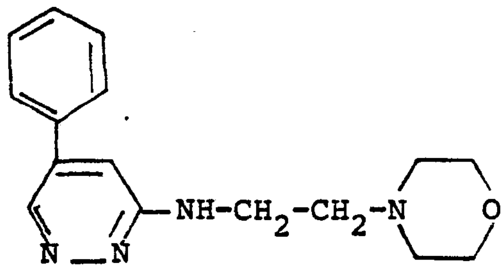
(I)

and its pharmaceutically acceptable salts.

2. Process for preparing the product of formula I, characterized in that the 3-chloro 5-phenyl pyridazine of formula II

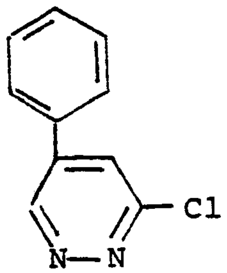
(II)

is reacted with the 2-morpholino ethylamine of formula III

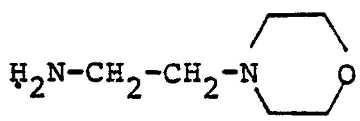
(III)

the reaction being carried out in an organic solvent, such as an alcohol, at a temperature ranging from about 50°C to the boiling temperature of said solvent, then optionally converting the product thus obtained into one of its pharmaceutically acceptable salts.

3. Process according to claim 2, characterized in that the reaction is carried out in the presence of copper used as catalyst.

4. Pharmaceutical compositions particularly useful in the treatment of depressive conditions and in the treatment of memory disorders, characterized in that they contain a compound according to claim 1.

5. Pharmaceutical compositions according to claim 4, characterized in that they contain 0.01 to 150 mg of the compound according to claim 1.

**Claims** for the contracting state: AT

1. Process for obtaining the derivative of 3. (2-morpholino ethylamino) 5-phenyl pyridazine of formula I

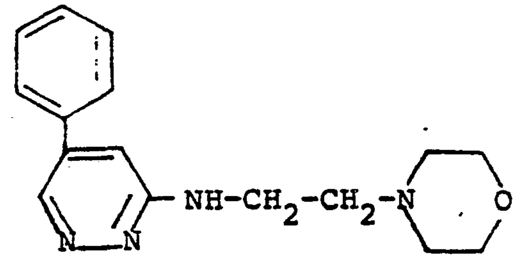
(I)

and of its pharmaceutically acceptable salts, characterized in that the 3-chloro 5-phenyl pyridazine of formula II

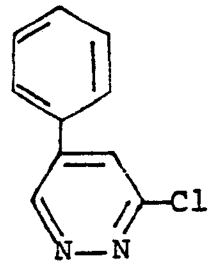
(II)

is reacted with the 2-morpholino ethylamine of formula III

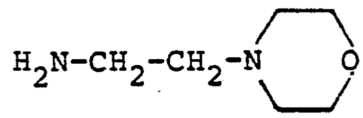
(III)

the reaction being carried out in an organic solvent, such as an alcohol, at a temperature ranging from about 50°C to the boiling temperature of said solvent, then optionally converting the product thus obtained into one of its pharmaceutically acceptable salts.

2. Process according to claim 1, characterized in that the reaction is carried out in the presence of copper used as catalyst.

3. Use of a compound according to claim 1, for preparing a drug particularly useful in the treatment of depressive conditions and in the treatment of memory disorders.

4. Use according to claim 3, characterized in that said drug contains 0.01 to 150 mg of a compound according to claim 1.